# EUROPEAN PATENT SPECIFICATION

(11) **EP 1 384 068 B1**
(45) Date of publication and mention of the grant of the patent: **17.09.2008**
(21) Application number: 02729125.1
(22) Date of filing: 02.05.2002
(51) Int. Cl.: G01N 27/26, G01N 33/53, G01N 33/537, G01N 33/547

(54) **METHODS FOR DETERMINING SECONDARY MODIFICATIONS OF MOLECULES USING ARRAYS**
VERFAHREN ZUR BESTIMMUNG SEKUNDÄRER MOLEKÜLMODIFIKATIONEN UNTER VERWENDUNG VON ARRAYS
METHODES PERMETTANT DE DETERMINER DES MODIFICATIONS SECONDAIRES DE MOLECULES AU MOYEN DE JEUX ORDONNES

(30) Priority: 02.05.2001 US 288285 P
(43) Date of publication of application: 28.01.2004
(73) Proprietor: Sigma-Genosys, Ltd., The Woodlands, TX 77380-3600 (US)
(72) Inventor: GILMORE, James c/o Sigma-Aldrich Co, St. Louis, Missouri 63103 (US); DANIEL, Steven, The Woodlands, TX 77382 (US); WIESE, Rick, The Woodlands, TX 77385 (US)
(74) Representative: Ratcliffe, Susan Margaret
(86) International application number: PCT/US2002/014043
(87) International publication number: WO 2002/088324

(56) References cited:
- WO-A-02/14860
- US-A- 4 469 787
- US-A- 5 482 867
- US-A- 5 545 531
- US-A- 5 885 837
- US-A- 5 891 656
- HUANG JOE ET AL: "The use of high-throughput, medium density microarrays for genotyping analysis" HUMAN MUTATION, vol. 17, no. 4, 2001, pages 333-334, XP009032151 Third International Meeting on Single Nucleotide Polymorphism and Complex Genome Analysis;Taos, New Mexico, USA; September 08-11, 2000 ISSN: 1059-7794
- HOGAN MICHAEL ET AL: "High-throughput, medium-density microarray technology to rapidly screen thousands of samples" INTERNATIONAL GENOME SEQUENCING AND ANALYSIS CONFERENCE, vol. 12, 2000, page 66 XP001181649 12th International Genome Sequencing and Analysis Conference;Miami Beach, Florida, USA; September 12-15, 2000
- BELOSLUDTSEV YURI ET AL: "DNA microarrays based on noncovalent oligonucleotide attachment and hybridization in two dimensions" ANALYTICAL BIOCHEMISTRY, vol. 292, no. 2, 17 April 2001 (2001-04-17) - 15 May 2001 (2001-05-15), pages 250-256, XP002284874 ISSN: 0003-2697
- HUANG JOE XI ET AL: "High-throughput genomic and proteomic analysis using microarray technology" CLINICAL CHEMISTRY, vol. 47, no. 10, October 2001 (2001-10), pages 1912-1916, XP002284875 ISSN: 0009-9147
- TAM S W ET AL: "Simultaneous analysis of eight human Th1/Th2 cytokines using microarrays" JOURNAL OF IMMUNOLOGICAL METHODS, ELSEVIER SCIENCE PUBLISHERS B.V.,AMSTERDAM, NL, vol. 261, no. 1-2, 1 March 2002 (2002-03-01), pages 157-165, XP004341276 ISSN: 0022-1759
- WIESE R ET AL: "Simultaneous multianalyte ELISA performed on a microarray platform" CLINICAL CHEMISTRY, AMERICAN ASSOCIATION FOR CLINICAL CHEMISTRY. WINSTON, US, vol. 47, no. 8, August 2001 (2001-08), pages 1451-1457, XP002254091 ISSN: 0009-9147
- BOULIKAS TENI: "Control of DNA replication by protein phosphorylation" ANTICANCER RESEARCH, vol. 14, no. 6B, 1994, pages 2465-2472, XP009032335 ISSN: 0250-7005

## Description

### TECHNICAL FIELD

This invention relates generally to cell biology, proteomics and polypeptide array, or "biochip," technology. In particular, the invention is directed to methods for measuring and analyzing secondary modifications, such as "post-translational" modifications, of a molecule using an array, e.g., a protein microarray.

### BACKGROUND

Biological molecules, including proteins and polysaccharides, are often modified (e.g., post-translationally modified) to activate, inactivate, or target the molecule. These states can be biologically relevant. For example, phosphorylation of the polypeptide p53, a major cell cycle regulator, regulates its activity. The activity of MAPK, an activator of signal transduction pathways, is also modified by phosphorylation. Another example is ARPP-21, a cyclic AMP-regulated phosphoprotein of M(r) 21 kDa that is enriched in the cell bodies and terminals of neurons in the basal ganglia. Using a phosphorylation state-specific antibody selective for the detection of ARPP-21 phosphorylated on Ser(55), activation of dopamine D1 receptors can be demonstrated; activation of D2 receptors causes a decrease in ARPP-21 phosphorylation (see, e.g., Caporaso (2000) Neuropharmacology 39:1637-1644).

By measuring secondary modifications of biological molecules, e.g., post-translational modifications of proteins, researchers can gain an understanding of disease, uncover potential diagnostic markers, and generate targets for therapeutics.

### SUMMARY

The invention provides a method for detecting a secondary modification of a target molecule using arrays. Use of microarrays allows for simultaneous analysis and detection of multiple secondary modification characteristics of a sample analyte, e.g., a post-translationally modified polypeptide. The invention provides a method for detecting a secondary modification of a target molecule comprising the following steps: (a) providing an array comprising a plurality of biosites, each biosite comprising a plurality of capture probes immobilized to the substrate; (b) providing a target molecule; (c) providing a detection probe capable of specifically binding to a capture probe-bound target molecule, wherein the detection probe specifically binds to the target probe; and, (d) contacting the target molecule with the array and the detection probe with the target molecule and detecting which biosite comprises a bound target molecule and detection probe, thereby detecting a secondary modification of the target molecule.

In one aspect of the methods of the invention, the secondary modification comprises a phosphorylation. The target molecule can comprise a polypeptide (including peptides, peptidomimetics, and the like). The secondary modification can comprise a post-translational modification. The secondary modification can comprise a phosphorylation of an amino acid residue; the amino acid residue can be selected from the group consisting of a serine, a tyrosine and a threonine. The secondary modification can comprise addition of a lipid moiety to an amino acid residue. The secondary modification can comprise addition of a saccharide moiety to an amino acid residue.

In an alternative aspect of the methods of the invention, the target molecule can be selected from the group consisting of a lipid, a nucleic acid and a carbohydrate. The carbohydrate can be a polysaccharide. The capture probe can comprise a polypeptide. The polypeptide can comprise an antibody. The capture probe can comprise a small molecule. The capture probe can comprise an array-immobilized nucleic acid hybridized to a chimeric molecule comprising two domains, wherein the first domain hybridizes specifically to the array-immobilized nucleic acid and the second domain specifically binds to the target molecule.

In an alternative aspect of the methods of the invention, the detection probe comprises a detectable moiety selected from the group consisting of a radioactive moiety, a colorimetric moiety, a bioluminescent moiety, a fluorescent moiety and a chemiluminescent moiety.

In one aspect, detecting which biosite includes a bound target molecule and detection probe comprises scanning the substrate surface and determining if any or sufficient detection probe has been immobilized to each biosite. The substrate surface scanning can be performed by an optical or an electrical device. In one aspect, the substrate surface can comprise a biosite comprising a capture probe capable of detecting an unmodified molecule and a biosite comprising a capture probe capable of detecting a modified molecule. The method can comprise use of a detection probe capable of detecting an unmodified molecule and a detection probe capable of detecting a modified molecule.

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure 1 schematically sets forth the specificity of the biosites on the array used in the exemplary methods described in Example 1.
Figure 2 is a representation of an image of an array demonstrating the specificity of anti-phospho-specific MAPK antibodies using the array format methods of the invention, as described in detail in Example 1, below.
Figure 3 is a graph representing data summarizing ERK1 phosphate concentrations and a standard curve, as described in detail in Example 1, below.

Like reference symbols in the various drawings indicate like elements.

### DETAILED DESCRIPTION

This invention provides methods for the simultaneous detection of multiple secondary modifications, such as "post-translational modifications," of molecules in a sample utilizing an "array" or "microarray" format. This is accomplished through the use of a secondary reporter molecule that specifically recognizes a modified epitope of a target molecule" (i.e., the antigen) while the target molecule is bound to the array. The modification can be, e.g., a phosphorylation or a glycosylation of a polypeptide, or, a modification (e.g., trimming) of a branched polysaccharide. The post-translational modification can be by a natural cellular process, or, it can be by an induced process, e.g., by a drug, a carcinogen, irradiation, induced reduction or oxidation and the like.

The format of the microarray allows simultaneous analysis (screening) of multiple antigens (as analytes) for secondary changes in structure or form, e.g., post-translational modifications. Further, by applying this format to microarrays, multiple characteristics of a sample analyte can be determined simultaneously, such as amount analyte present, conformation and modification status.

### Definitions

Unless defined otherwise, all technical and scientific terms used herein have the meaning commonly understood by a person skilled in the art to which this invention belongs. As used herein, the following terms have the meanings ascribed to them unless specified otherwise.

The terms "array" or "microarray" or "protein array" or "proteome array" or "biochip" as used herein are used interchangeably herein, and include all known variations of these devices, as discussed in detail, below.

By "biosite" is meant the biological molecules or capture probes that are deposited on the top surface of the reaction substrate, or base material. Under appropriate conditions, an association, e.g., a specific binding, or hybridization, can occur between the probe and a target molecule. The component strands of the biological molecule form the biosite since there is the potential of an interaction or reaction occurring between component strands of the biological molecule and the target molecule(s). The maximum number of biosites per array will depend on the size of the array, or reaction vessel within an array, may vary, depending on the probe deposition technology (e.g., printing), the nature of the probe, the means used to assess binding and/or to determine the volume or shape of a biosite (for quality control). For example, the size of a biosite on an array may depend on the practical optical resolution of the accompanying detector/imager. For example, an array of 16 (4 × 4 array) biosites may be deposited on the hybridization substrate or base material that eventually forms the bottom of the entire reaction vessel. In this example, each biosite may comprise a circle of approximately about 25 to 200 microns (µm) in diameter. Thus, for a 16 biosite array, each of the 16 × 200 µm diameter area contains a uniform field of probes attached to the hybridization substrate (base material) in a concentration which is highly dependent on the probe size and the well size. Each 25 to 200 µm diameter area can contain millions of probe molecules. Also, each of the 16 different biosites (probe sites) can contain one type of probe. Thus, 16 different probe types can be assayed in an array containing 16 biosites (4 × 4 array) per reaction chamber. As another example, four separate 10×10 arrays (400 biosites) can be generated to fit into one well of a 96 well microtiter plate with sufficient spacing between each of the 400 biosites. For this 10×10 format, 400 hybridization experiments are possible within a single reaction chamber corresponding to 38,400 (96 × 400) assays/hybridization that can be performed nearly simultaneously.

By "substrate" is meant the substrate that the biosites, or probes, are deposited. "Substrates" can be selected from a variety of materials, without limitation, e.g., polyvinyl, polystyrene, polypropylene, polyester, vinyl, other plastics, glass, SiO₂, other silanes, nylon membrane, gold or platinum, see further examples described, below. The solid surfaces can be derivatized, e.g., thiol-derivatized biopolymers and organic thiols can be bound to a metal solid substrate; see, e.g., U.S. Patent No. 5,942,397 (see below for more examples).

The term "immobilized" means that the probe can be attached to a surface (e.g., the substrate) in any manner or any method; including, e.g., reversible or non-reversible binding, covalent or non-covalent attachment, and the like.

The term "detection probe" means any molecule that can be directly or indirectly detected by any means, including electronic or visual methods; thus, the detection probe can comprise two molecules, including a first molecule that specifically binds the target probe and a second molecule that binds the first molecule. In one embodiment, the detection probe comprises the target molecule, e.g., the target molecule is a polypeptide phosphorylated with radioactive P³² (and the capture molecule binds to an epitope comprising the post-translational modification); see, e.g., U.S. Patent No. 5,538,858.

The term "dynamic range" means the difference between the most and least sensitive signal. The term "specificity" means the ability of a molecule (e.g., a protein or small molecule) to recognize and differentiate a second molecule (by "specifically binding to the second molecule). The term "sensitivity" means the minimum signal that can be recognized above background signal. The term "background" means the signal generated by noise and/or non-specific binding. For example, background can be determined where a capture antibody has not been printed onto an array. The term "degradation" means the loss of structural confirmation in a protein, for example as through a deletion or alteration in the amino acid sequence. The term "markers" means capture antibodies that are printed in a pattern such that the orientation can be easily recognized. The term "housekeepers" means antigens present in sample that are believed to vary little in concentration or composition from sample to sample. The term "solution" means a liquid or semi-liquid that is comprised of varying buffers and/or sample and is applied to the protein array.

The term "antibody" refers to a peptide or polypeptide substantially encoded by an immunoglobulin gene or immunoglobulin genes, or fragments or equivalents thereof, capable of specifically binding an epitope, see, e.g. Fundamental Immunology, Third Edition, W.E. Paul, ed., Raven Press, N.Y. (1993); Wilson (1994) J. Immunol. Methods 175:267-73; Yarmush (1992) J. Biochem. Biophys. Methods 25:85-97. One of skill will appreciate that antibody fragments may be isolated or synthesized *de novo* either chemically or by utilizing recombinant DNA methodology. The term antibody also includes "chimeric" antibodies either produced by the modification of whole antibodies or those synthesized *de novo* using recombinant DNA methodologies. Typically, such chimeric antibodies are "humanized antibodies," *i.e.*, where the epitope binding site is generated from an immunized mammal, such as a mouse, and the structural framework is human. Immunoglobulins can also be generated using phage display libraries, and variations thereof. Antibodies or other molecules that bind to post-translationally modified polypeptides are well known in the art, see, e.g., U.S. Patent No. 6,008,024; 5,763,198; 5,599,681; 5,580,742.

### Nucleic Acid and Polypeptide Probes

This invention provides an array comprising immobilized capture molecules, which can be immobilized polypeptides, nucleic acids or oligonucleotide (and polysaccharides or small molecules). The "detection probes" can also be polypeptides, nucleic acids or oligonucleotides (and polysaccharides or small molecules). For example, a polypeptide can be immobilized to an array substrate surface by conjugation to an oligonucleotide, which in turn specifically hybridizes to a nucleic acid immobilized on the array surface (see, e.g., U.S. Patent No. 6,083,763). These probes can be made and expressed *in vitro* or *in vivo,* any means of making and expressing polypeptides or nucleic acids used in the devices or practiced with the methods of the invention can be used. The invention can be practiced in conjunction with any method or protocol known in the art, which are well described in the scientific and patent literature.

The nucleic acids of the invention, e.g., probes of the arrays, whether, e.g., RNA, cDNA, fragments of genomic DNA, can be isolated from a variety of sources, genetically engineered, amplified, and/or expressed recombinantly. Any recombinant expression system can be used, including, in addition to mammalian cells, e.g., bacterial, yeast, insect or plant systems. Alternatively, these nucleic acids can be synthesized *in vitro* by well-known chemical synthesis techniques, as described in, e.g., Carruthers (1982) Cold Spring Harbor Symp. Quant. Biol. 47:411-418; Belousov (1997) Nucleic Acids Res. 25:3440-3444; Frenkel (1995) Free Radic. Biol. Med. 19:373-380; Blommers (1994) Biochemistry 33:7886-7896; Brown (1979) Meth. Enzymol. 68:109; Beaucage (1981) Tetra. Lett. 22:1859; U.S. Patent No. 4,458,066.

Techniques for the manipulation of nucleic acids, such as, e.g., generating mutations in sequences, subcloning, labeling probes, sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Capture probes and detection probes can include, e.g., amino acids, peptides, oligopeptide, polypeptides, peptidomimetics, other short polymers or organic molecules. When amino acids are used, alternative embodiment can use methyl esters because of commercial availability and the fact that they are not altered by the formation reactions (binding of the association surface to the support surface). "Peptidomimetics" include synthetic chemical compounds that have substantially the same structural and/or functional characteristics of the corresponding composition, e.g., the peptides, oligopeptides (e.g., oligo-histidine, oligo-aspartate, oligo-glutamate, poly-(his)₂(gly)₁, and poly-(his)₂(asp)₁), polypeptides, imidazole derivatives or equivalents used in the association surface of the invention. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative substitutions as long as such substitutions also do not substantially alter the mimetic's structure and/or activity. Individual peptidomimetic residues can be joined by peptide bonds, other chemical bonds or coupling means, such as, e.g., glutaraldehyde, N-hydroxysuccinimide esters, bifunctional maleimides, N,N'-dicyclohexylcarbodiimide (DCC) or N,N'-diisopropyl-carbodiimide (DIC). Linking groups that can be an alternative to the traditional amide bond ("peptide bond") linkages include, e.g., ketomethylene (e.g., -C(=O)-CH₂- for -C(=O)-NH-), aminomethylene (CH₂-NH), ethylene, olefin (CH=CH), ether (CH₂-O), thioether (CH₂-S), tetrazole (CN₄-), thiazole, retroamide, thioamide, or ester (see, e.g., Spatola (1983) in Chemistry and Biochemistry of Amino Acids, Peptides and Proteins, Vol. 7, p 267-357, Marcell Dekker, NY).

### Arrays, or "BioChips"

The invention provides methods for simultaneous analysis (screening) of multiple antigens (as analytes) for secondary modifications, such as "post-translational" modifications. Arrays used in the methods of the invention comprise a plurality of target elements or "capture probes," each immobilized target element comprising a defined amount of one or more polypeptide or nucleic acid molecule, or probes. The capture probes are immobilized onto a solid surface for binding (directly or indirectly) to a target molecule, which, in the methods of this invention, is the molecule to be analyzed for the presence of post-translational modifications. The biosites may be arranged on the solid surface at different sizes and different densities. The methods of the invention can incorporate in whole or in part designs of arrays, and associated components and methods, as described, e.g., in U.S. Patent Nos. 6,197,503; 6,174,684; 6,156,501; 6,093,370; 6,087,112; 6,087,103; 6,087,102; 6,083,697; 6,080,585; 6,054,270; 6,048,695; 6,045,996; 6,022,963; 6,013,440; 5,959,098; 5,856,174; 5,843,655; 5,837,832; 5,770,456; 5,723,320; 5,700,637; 5,695,940; 5,556,752; 5,143,854; see also, e.g., WO 99/51773; WO 99/09217; WO 97/46313; WO 96/17958; WO 89/10977; see also, e.g., Johnston (1998) Curr. Biol. 8:R171-R174; Schummer (1997) Biotechniques 23:1087-1092; Kern (1997) Biotechniques 23:120-124; Solinas-Toldo (1997) Genes, Chromosomes & Cancer 20:399-407; Bowtell (1999) Nature Genetics Supp. 21:25-32; Epstein (2000) Current Opinion in Biotech. 11:36-41; Mendoza (1999) "High-throughput microarray-based enzyme-linked immunosorbent assay (ELISA)," Biotechniques 27: 778-788; Lueking (1999) Protein microarrays for gene expression and antibody screening," Anal. Biochem. 270:103-111; Davies (1999) "Profiling of amyloid beta peptide variants using SELDI protein chip arrays," Biotechniques 27:1258-1261.

### Probe deposition onto substrate

The invention provides for making an array by immobilizing onto a substrate a plurality of biosites comprising "capture probes." The probes can be "deposited" or immobilized" onto the substrate using any method or combination of methods known in the art, e.g., pizo-electric, such as ink-jet, processes and systems, robotic deposition, photolithographic in-situ synthesis, use of microsyringes, or a continuous flow bundled microcapillary process (see, e.g., U.S. Patent No. 6,083,763). Array fabrication methods that can be incorporated, in whole or in part, in the making or using of the invention include, e.g., those described in U.S. Patent Nos. 6,197,503; 6,177,238; 6,164,850; 6,150,147; 6,083,763; 6,048,695; 6,010,616; 5,599,695; 5,919,523; 5,861,242; 5,770,722; 5,750,669; 5,143,854.

### Substrate Surfaces

The arrays used in the methods of the invention can comprise substrate surfaces of a rigid, semi-rigid or flexible material. The substrate surface can be flat or planar, be shaped as wells, raised regions, etched trenches, pores, beads, filaments, or the like. Substrates can be of any material upon which a "capture probe" can be directly or indirectly bound. For example, suitable materials can include paper, glass (see, e.g., U.S. Patent No. 5,843,767), ceramics, quartz or other crystalline substrates (e.g. gallium arsenide), metals, metalloids, polacryloylmorpholide, various plastics and plastic copolymers, Nylon^{™}, Teflon^{™}, polyethylene, polypropylene, poly(4-methylbutene), polystyrene, polystyrene/ latex, polymethacrylate, poly(ethylene terephthalate), rayon, nylon, poly(vinyl butyrate), polyvinylidene difluoride (PVDF) (see, e.g., U.S. Patent No. 6,024,872), silicones (see, e.g., U.S. Patent No. 6,096,817), polyformaldehyde (see, e.g., U.S. Patent Nos. 4,355,153; 4,652,613), cellulose (see, e.g., U.S. Patent No. 5,068,269), cellulose acetate (see, e.g., U.S. Patent No. 6,048,457), nitrocellulose, various membranes and gels (e.g., silica aerogels, see, e.g., U.S. Patent No. 5,795,557), paramagnetic or superparamagnetic microparticles (see, e.g., U.S. Patent No. 5,939,261) and the like. The substrate can be derivatized for application of other compounds upon which the probes are immobilized. Reactive functional groups can be, e.g., hydroxyl, carboxyl, amino groups or the like. Silane (e.g., mono- and dihydroxyalkylsilanes, aminoalkyltrialkoxy-silanes, 3-aminopropyl-triethoxysilane, 3-aminopropyltrimethoxysilane) can provide a hydroxyl functional group for reaction with an amine functional group.

### Detection Probes and Devices

The detection probe can comprise any detectable moiety, including, e.g., radioactive, colorimetric, bioluminescent, fluorescent or chemiluminescent or another photon detectable moieties. The detection probe also comprises any molecule that specifically binds to the target molecule when the target molecule is specifically bound to the capture probe. The detection probe can comprise a polypeptide, a lipid, a small molecule, a polysaccharide, a nucleic acid or a combination thereof. Fluorescence, bioluminescence or chemiluminescence, or radiation, can be detected and quantified, e.g., using assays and devices well known in the art, as described in, e.g., U.S. Patent Nos. 6,225,670; 6,211,524; 6,197,928; 6,197,499; 6,194,731; 6,194,223; 6,191,852; 6,191,425; 6,132,983; 6,087,476; 6,060,261; 5,866,348; 5,094,939; 5,744,320; 5,631,734; 5,192,980; 5,091,652.

The binding of the "detection probe" to the molecule to be analyzed for secondary modifications (e.g., post-translational modifications) can be performed in any manner using any detection device, e.g., by scanning the substrate surface and determining if any or sufficient detection probe has been bound to molecule affixed to a biosite on the substrate surface area. These functions can be performed by any device, e.g., an optical or an electrical device.

For example, one imaging system can be a proximal charge-coupled device (CCD) detection/imaging; due to its inherent versatility, it can also accommodate chemiluminescence, fluorescent and radioisotope target molecule detection, high throughput, and high sensitivity. This detection/imaging apparatus can include a lensless imaging array comprising a plurality of solid state imaging devices, such as an array of CCDs, photoconductor-on-MOS arrays, photoconductor-on-CMOS arrays, charge injection devices (CIDs), photoconductor on thin-film transistor arrays, amorphous silicon sensors, photodiode arrays, or the like.

The devices and methods of the invention incorporate in whole or in part designs of detection devices as described, e.g., in U.S. Patent Nos. 6,197,503; 6,197,498; 6,150,147; 6,083,763; 6,066,448; 6,045,996; 6,025,601; 5,599,695; 5,981,956; 5,698,089; 5,578,832; 5,632,957.

### EXAMPLES

The following examples are offered to illustrate, but not to limit the claimed invention.

### Example 1: Determining secondary structure modifications using an array format

Exemplary methods for practicing the methods of the invention are provided, including fabrication of protein microarrays and analysis of samples.

*Slide Preparation.* Standard well glass slides (e.g., GENOMETRIX 16^{™} glass slides) were cleaned and silanized. Arrays were printed on prepared slides with a capillary printer. Print solutions consisted of appropriate antigens diluted no less than 1:1 in print buffer (0.1 M carbonate buffer, pH 9.5 + 5% glycerol). The phosphorylated-MAPK and non-phosphorylated MAPK antigens (see, e.g., U.S. Patent Nos. 5,405,941 and 5,663,314) were purchased from Upstate Laboratories (Syracuse, NY) and printed in serial dilution. Anti-phosphospecific MAPK antibody was purchased from Upstate Laboratories. The positional and positive control marker used for these arrays was rabbit IgG (Fitzgerald Industries International, Inc, Concord, MA; #31-RGG0) used at a print concentration of 150 Tg/ml. The slides were visually inspected after printing for quality of print.

*Microassay.* After overnight storage at 4°C, the sample wells were first rinsed in triplicate then blocked on a shaker plate for one hour at room temperature in blocker casein (#37528ZZ, Pierce Chemical Co., Rockford, IL). Blocker casein was aspirated from the wells and appropriate antibody solutions, diluted in PBS, were added to each of the test wells. The array plate was placed in a humidity chamber and incubated at 37°C for 2 hours. After sample incubation, the plate was removed from the oven and washed 3 times with blocker casein. Biotinylated anti-sheep (Jackson Labs, Bar Harbor, Maine) antibody was then applied to every well. The biotinylated antibody was detected with ALEXA 546^{™} conjugated streptavidin (Molecular Probes, Eugene OR). The samples were once again incubated at 37°C in a humidity chamber, for an hour and a half.

The completed assay slide was imaged utilizing Packard LUMONICS^{™} Scanner. The saved TIFF images were finally analyzed utilizing software, (e.g., a custom dot scoring software, Genometrix Genomics, Inc., The Woodlands, TX). The software should automatically subtract background from the utilized densitometry values. Dot score values were used to construct a densitometry versus antigen concentration graph. Signal intensity from the phosphospecific antibody was plotted against the phosphorylated and non-phosphorylated MAPK.

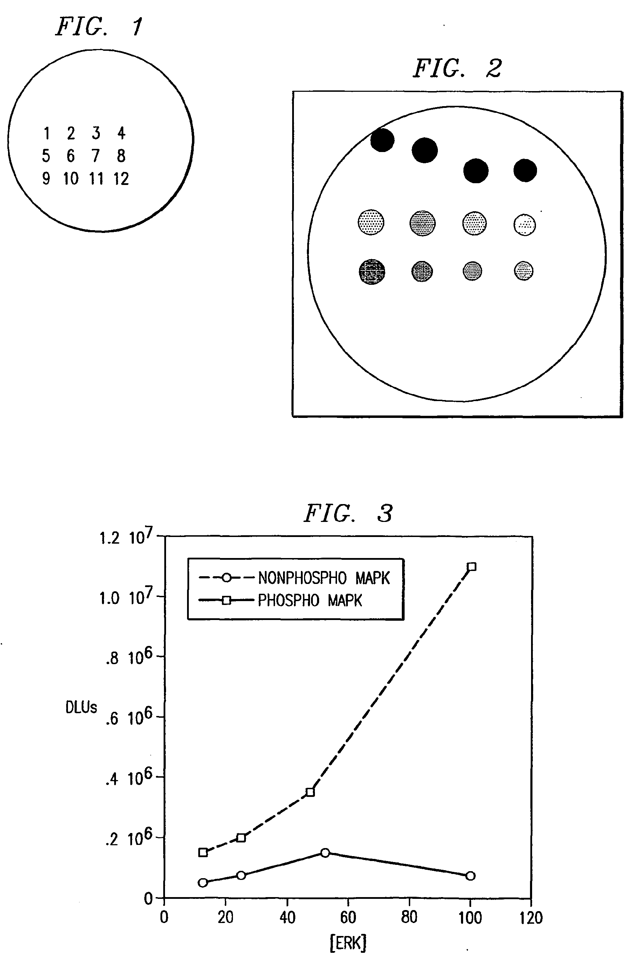

The specificity of the biosites on the array, or "array map," is set forth in Figure 1: markers **1-4-** sheep IgG marker 10 mcg/ml; marker **5-** 100 mcg/ml nonphospho ERK; marker **6-8-** 1:1 dilutions of 5; marker **9-** 100 mcg/ml phospho ERK; markers **10-12-** 1:1 dilution series of 9.

The array design incorporated a phosphate-bearing epitope and a non-phosphate-bearing epitope of ERK printed using a serial dilution (Figure 1). Anti-phosphate-specific MAPK antibody was applied to the array and imaged using direct fluorescence. Figure 2 is a representation of these fluorescent images from the array; the images demonstrate the specificity of anti-phospho-specific MAPK. The amount of signal intensity was determined for each element on the array. Figure 3 is a graph representing data summarizing ERK1 phosphate concentrations and a standard curve.

The amount of discrimination between the two MAPK epitopes was dependent upon the concentration, with the higher concentration of phospho-MAPK giving higher signal intensity and thus greater resolution. However, the two epitopes could be discriminated at all ranges tested. Thus, these experiments demonstrate that the methods of the invention, using microarray platforms, can discriminate specific epitopes of antigens.

## Claims

1. A method for detecting a secondary modification of a target molecule comprising the following steps:
(a) providing an array comprising a plurality of biosites, one or more of the biosites including a plurality of capture probes immobilized to a substrate surface;
(b) contacting a target molecule with the array, or the array with the target molecule
(c)contacting a detection probe with the target molecule, or the target molecule with the detection probe and
(d)detecting which biosite comprises a bound target molecule and detection probe.

2. A method according to claim 1 wherein the detection probe is arranged to specifically bind to a capture probe-bound target molecule

3. A method according to any preceding claim, wherein the secondary modification comprises a phosphorylation.

4. A method according to claims 1 to 2 wherein the target molecule comprises a polypeptide.

5. A method according to claim 4, wherein the secondary modification comprises a post-translational modification, or a phosphorylation of an amino acid residue.

6. A method according to claim 5, wherein the amino acid residue is selected from the group consisting of a serine, a tyrosine and a threonine.

7. A method according to claim 5, wherein the secondary modification comprises an addition of a lipid moiety to an amino acid residue, or an addition of a saccharide moiety to an amino acid residue.

8. A method according to claims 1 to 2 wherein the target molecule is selected from the group consisting of a lipid, a nucleic acid and a carbohydrate (such as a polysaccharide).

9. A method according to any preceding claim; wherein the capture probe comprises a polypeptide (which preferably comprises an antibody), or comprises a small molecule.

10. A method according to claims 1 to 2 wherein the capture probe comprises an array-immobilized nucleic acid hybridized to a chimeric molecule comprising two domains, wherein the first domain hybridizes specifically to the array-immobilized nucleic acid and the second domain specifically binds to the target molecule.

11. A method according to any preceding claim, wherein the detection probe includes a detectable moiety selected from the group consisting of a radioactive moiety, a colorimetric moiety, a bioluminescent moiety, a fluorescent moiety and a chemiluminescent moiety.

12. A method according to any preceding claim, wherein detecting which biosite includes an immobilized target molecule and detection probe comprises scanning the substrate surface and determining if the detection probe is immobilized at the biosites

13. A method according to of claim 12, wherein the scanning is performed by an optical or an electrical device.

14. A method according to any preceding claim, wherein the substrate surface comprises a biosite including a capture probe capable of detecting an unmodified molecule.

15. A method according to any preceding claim, wherein the substrate surface comprises a biosite including a capture probe capable of detecting a modified molecule

16. A method according to any preceding claim, wherein providing a detection probe comprises use of a detection probe capable of detecting an unmodified molecule and a detection probe capable of detecting a modified molecule.

## Patentansprüche

1. Verfahren zum Detektieren einer sekundären Modifikation eines Target-Moleküls, umfassend die folgenden Schritte:
(a) Vorsehen eines Arrays, umfassend eine Vielzahl von Biosites, wobei einer oder mehrere der Biosites eine Vielzahl von Bindungssonden enthalten, die auf einer Substratoberfläche immobilisiert sind,
(b) Kontaktieren eines Target-Moleküls mit dem Array oder den Array mit dem Target-Molekül,
(c) Kontaktieren einer Detektionssonde mit dem Target-Molekül oder das Target-Molekül mit der Detektionssonde und
(d) Detektieren, welcher Biosite ein gebundenes Target-Molekül und eine Detektionssonde umfasst.

2. Verfahren nach Anspruch 1, wobei die Detektionssonde so eingerichtet ist, dass sie spezifisch an ein Target-Molekül bindet, an das eine Bindungssonde gebunden ist.

3. Verfahren nach einem der vorangehenden Ansprüche, wobei die sekundäre Modifikation eine Phosphorylierung umfasst.

4. Verfahren nach Anspruch 1 oder 2, wobei das Target-Molekül ein Polypeptid umfasst.

5. Verfahren nach Anspruch 4, wobei die sekundäre Modifikation eine posttranslationale Modifikation oder eine Phosphorylierung eines Aminosäurerestes umfasst.

6. Verfahren nach Anspruch 5, wobei der Aminosäurerest ausgewählt ist aus der Gruppe bestehend aus Serin, Tyrosin und Threonin.

7. Verfahren nach Anspruch 5, wobei die sekundäre Modifikation die Addition einer Lipid-Gruppe an einen Aminosäurerest oder die Addition einer Saccharid-Gruppe an einen Aminosäurerest umfasst.

8. Verfahren nach Anspruch 1 oder 2, wobei das Target-Molekül ausgewählt ist aus der Gruppe bestehend aus Lipid, Nukleinsäure und Kohlenwasserstoff (wie z. B. Polysaccharid).

9. Verfahren nach einem der vorangehenden Ansprüche, wobei die Bindungssonde ein Polypeptid umfasst (welches bevorzugt einen Antikörper umfasst) oder ein kleines Molekül umfasst.

10. Verfahren nach Anspruch 1 oder 2, wobei die Bindungssonde eine Arrayimmobilisierte Nukleinsäure umfasst, die hybridisiert ist mit einem chimären Molekül, umfassend zwei Domains, wobei die erste Domain spezifisch mit der Array-immobilisierten Nukleinsäure hybridisiert und die zweite Domain spezifisch an das Target-Molekül bindet.

11. Verfahren nach einem der vorangehenden Ansprüche, wobei die Detektionssonde eine detektierbare Gruppe umfasst, ausgewählt aus der Gruppe bestehend aus radioaktive Gruppe, kolorimetrische Gruppe, biolumineszente Gruppe, fluoreszente Gruppe und chemilumineszente Gruppe.

12. Verfahren nach einem der vorangehenden Ansprüche, wobei das Detektieren, welcher Biosite ein immobilisiertes Target-Molekül und eine Detektionssonde enthält, das Scannen der Substratoberfläche und das Bestimmen, ob die Detektionssonde an dem Biosite immobilisiert ist, umfasst.

13. Verfahren nach Anspruch 12, wobei das Scannen durchgeführt wird durch eine optische oder eine elektrische Vorrichtung.

14. Verfahren nach einem der vorangehenden Ansprüche, wobei die Substratoberfläche einen Biosite umfasst, enthaltend eine Bindungssonde, die in der Lage ist, ein unmodifiziertes Molekül zu detektieren.

15. Verfahren nach einem der vorangehenden Ansprüche, wobei die Substratoberfläche einen Biosite umfasst, enthaltend eine Bindungssonde, die in der Lage ist, ein modifiziertes Molekül zu detektieren.

16. Verfahren nach einem der vorangehenden Ansprüche, wobei das Vorsehen einer Detektionssonde die Verwendung einer Detektionssonde, die in der Lage ist, ein unmodifiziertes Molekül zu detektieren, und einer Detektionssonde, die in der Lage ist, ein modifiziertes Molekül zu detektieren, umfasst.

## Revendications

1. Procédé de détection d'une modification secondaire d'une molécule cible, comprenant les étapes suivantes :
(a) élaboration d'un réseau comprenant une pluralité de biosites, un ou plusieurs des biosites incluant une pluralité de sondes de capture immobilisées sur une surface de substrat ;
(b) mise en contact d'une molécule cible avec le réseau ou du réseau avec la molécule cible ;
(c) mise en contact d'une sonde de détection avec la molécule cible ou de la molécule cible avec la sonde de détection et
(d) détection du biosite qui comprend une molécule cible liée et une sonde de détection.

2. Procédé selon la revendication 1, dans lequel la sonde de détection est adaptée de manière à se lier spécifiquement à une molécule cible liée à la sonde de capture.

3. Procédé selon l'une quelconque des revendications précédentes, dans lequel la modification secondaire comprend une phosphorylation.

4. Procédé selon l'une des revendications 1 ou 2, dans lequel la molécule cible comprend un polypeptide.

5. Procédé selon la revendication 4, dans lequel la modification secondaire comprend une modification post-traductionnelle ou une phosphorylation d'un résidu d'acide aminé.

6. Procédé selon la revendication 5, dans lequel le résidu d'acide aminé est choisi dans le groupe consistant en une sérine, une tyrosine et une thréonine.

7. Procédé selon la revendication 5, dans lequel la modification secondaire comprend une addition d'un groupement lipidique à un résidu d'acide aminé ou une addition d'un groupement saccharidique à un résidu d'acide aminé.

8. Procédé selon l'une des revendications 1 ou 2, dans lequel la molécule cible est choisie dans le groupe consistant en un lipide, un acide nucléique et un hydrate de carbone (tel qu'un polysaccharide).

9. Procédé selon l'une quelconque des revendications précédentes, dans lequel la sonde de capture comprend un polypeptide (comprenant de préférence un anticorps) ou comprenant une petite molécule.

10. Procédé selon l'une des revendications 1 ou 2, dans lequel la sonde de capture comprend un acide nucléique immobilisé sur un réseau et hybridé à une molécule chimérique comprenant deux domaines, le premier domaine s'hybridant spécifiquement à l'acide nucléique immobilisé sur le réseau et le second domaine se liant spécifiquement à la molécule cible.

11. Procédé selon l'une quelconque des revendications précédentes, dans lequel la sonde de détection inclut un groupement détectable choisi dans le groupe consistant en un groupement radioactif, un groupement colorimétrique, un groupement bioluminescent, un groupement fluorescent et un groupement chimioluminescent.

12. Procédé selon l'une quelconque des revendications précédentes, dans lequel la détection du biosite incluant une molécule cible immobilisée et la sonde de détection comprenant le criblage de la surface du substrat et le fait de déterminer si la sonde de détection est immobilisée au niveau des biosites.

13. Procédé selon la revendication 12, dans lequel le criblage est réalisé par un dispositif optique ou électrique.

14. Procédé selon l'une quelconque des revendications précédentes, dans lequel la surface du substrat comprend un biosite incluant une sonde de capture apte à détecter une molécule non modifiée.

15. Procédé selon l'une quelconque des revendications précédentes, dans lequel la surface du substrat comprend un biosite incluant une sonde de capture apte à détecter une molécule modifiée.

16. Procédé selon l'une quelconque des revendications précédentes, dans lequel la fourniture d'une sonde de détection comprend l'utilisation d'une sonde de détection apte à détecter une molécule non modifiée et d'une sonde de détection apte à détecter une molécule modifiée.
